# FASCICULE DE BREVET EUROPEEN

(11) **EP 2 872 626 B1**
(45) Date de publication et mention de la délivrance du brevet: **17.05.2017**
(21) Numéro de dépôt: 13731388.8
(22) Date de dépôt: 05.06.2013
(51) Int. Cl.: C12N 9/42, C12N 9/96, C12N 1/00, C12P 7/10

(54) **UTILISATION DU CO2 POUR LA DÉSACTIVATION D'UN MICROORGANISME CELLULOLYTIQUE UTILISÉ DANS LA CONVERSION BIOCHIMIQUE DE MATÉRIAUX LIGNO-CELLULOSIQUES**
VERWENDUNG VON CO2 ZUR DEAKTIVIERUNG EINES CELLULOLYTISCHEN MIKROORGANISMUS ZUR VERWENDUNG BEI DER BIOCHEMISCHEN UMWANDLUNG VON LIGNOCELLULOSEMATERIALIEN
USE OF CO2 FOR DEACTIVATING A CELLULOLYTIC MICROORGANISM USED IN THE BIOCHEMICAL CONVERSION OF LIGNOCELLULOSIC MATERIALS

(30) Priorité: 18.06.2012 FR 1201732
(43) Date de publication de la demande: 20.05.2015
(73) Titulaire: IFP Énergies nouvelles, 92852 Rueil-Malmaison Cedex (FR)
(72) Inventeur: BEN CHAABANE, Fadhel, F-75020 Paris (FR); LOURET, Sylvain, F-69003 Lyon (FR)
(86) Numéro de dépôt international: PCT/FR2013/051269
(87) Numéro de publication internationale: WO 2013/190203

(56) Documents cités:
- Mitchinson C: "Improved cellulases for the biorefinery: a review of genencor's progress in the DOE subcontract for cellulase cost reduction for bioethanol.", Stanford GCEP Biomass Energy Workshop, April 2004 , avril 2004 (2004-04), XP002695831, Extrait de l'Internet: URL:http://gcep.stanford.edu/pdfs/energy_w orkshops_04_04/biomass_mitchinson.pdf [extrait le 2013-04-15]
- SCHELL D J ET AL: "WHOLE BROTH CELLULASE PRODUCTION FOR USE IN SIMULTANEOUS SACCHARIFICATION AND FERMENTATION", APPLIED BIOCHEMISTRY AND BIOTECHNOLOGY, HUMANA PRESS, INC, UNITED STATES, vol. 24-25, no. SPRING-SUMMER, 1990, pages 287-298, XP008122849, ISSN: 0273-2289, DOI: 10.1007/BF02920253
- ELWYN T. REESE ET AL: "Stability of the Cellulase ofTrichoderma reesei under use conditions", BIOTECHNOLOGY AND BIOENGINEERING, vol. 22, no. 2, 1980, pages 323-335, XP055058050, ISSN: 0006-3592, DOI: 10.1002/bit.260220207
- CARDONA ET AL: "Fuel ethanol production: Process design trends and integration opportunities", BIORESOURCE TECHNOLOGY, ELSEVIER BV, GB, vol. 98, no. 12, 19 mars 2007 (2007-03-19) , pages 2415-2457, XP005932845, ISSN: 0960-8524, DOI: 10.1016/J.BIORTECH.2007.01.002
- KARA K PODKAMINER ET AL: "Ethanol and anaerobic conditions reversibly inhibit commercial cellulase activity in thermophilic simultaneous saccharification and fermentation (tSSF)", BIOTECHNOLOGY FOR BIOFUELS, vol. 5, no. 1, 15 juin 2012 (2012-06-15), page 43, XP055058045, ISSN: 1754-6834, DOI: 10.1073/pnas.0605381103
- M. MCINTYRE ET AL: "Dissolved carbon dioxide effects on morphology, growth, and citrate production in Aspergillus niger A60", ENZYME AND MICROBIAL TECHNOLOGY, vol. 20, no. 2, février 1997 (1997-02), pages 135-142, XP055058041, ISSN: 0141-0229, DOI: 10.1016/S0141-0229(96)00108-1
- M. MCINTYRE ET AL: "Morphogenetic and biochemical effects of dissolved carbon dioxide on filamentous fungi in submerged cultivation", APPLIED MICROBIOLOGY AND BIOTECHNOLOGY, vol. 50, no. 3, 28 septembre 1998 (1998-09-28), pages 291-298, XP055060474, ISSN: 0175-7598, DOI: 10.1007/s002530051293

## Description

### DOMAINE DE L'INVENTION

La présente invention concerne la production des enzymes cellulolytiques et hémicellulolytiques, notamment dans le cadre de la production d'éthanol à partir de matériaux cellulosiques ou ligno-cellulosiques.

### ART ANTÉRIEUR

Depuis les années 1970, la transformation de matériaux ligno-cellulosiques en éthanol, après hydrolyse des polysaccharides constitutifs en sucres fermentescibles, a fait l'objet de très nombreux travaux. On peut citer par exemple les travaux de référence du National Renewable Energy Laboratory (Process Design and Economics for Biochemical Conversion of Lignocellulosic Biomass to Ethanol, Humbird et al., NREL/TP-5100-57764, May 2011).

Les matériaux ligno-cellulosiques sont des matériaux cellulosiques, c'est-à-dire constitués à plus de 90% poids de cellulose, et/ou lignocellulosiques, c'est-à-dire constitués de cellulose, d'hémicelluloses, qui sont des polysaccharides essentiellement constitués de pentoses et d'hexoses ainsi que de lignine, qui est une macromolécule de structure complexe et de haut poids moléculaire, composée d'alcools aromatiques reliés par des liaisons éther.

Le bois, les pailles, les rafles de maïs sont les matériaux ligno-cellulosiques les plus utilisés, mais d'autres ressources, cultures forestières dédiées, résidus de plantes alcooligènes, sucrières et céréalières, produits et résidus de l'industrie papetière et des produits de transformation des matériaux ligno-cellulosiques sont utilisables. Ils sont constitués pour la plupart d'environ 35 à 50 % de cellulose, de 20 à 30 % d'hémicellulose et de 15 à 25 % de lignine.

Le procédé de transformation biochimique des matériaux ligno-cellulosiques en éthanol comprend une étape de prétraitement physico-chimique, suivie d'une étape d'hydrolyse enzymatique utilisant un cocktail enzymatique, d'une étape de fermentation éthanolique des sucres libérés, et d'une étape de purification de l'éthanol.

La fermentation éthanolique et l'hydrolyse enzymatique, dite également saccharification, peuvent être conduites simultanément (Simultaneous Saccharification and Fermentation, ou
SSF selon la terminologie anglo-saxonne), par exemple en ajoutant des organismes éthanoliques au cours de l'étape d'hydrolyse.

Le cocktail enzymatique est un mélange d'enzymes cellulolytiques (également appelées cellulases) et/ou hémicellulolytiques. Les enzymes cellulolytiques présentent trois grands types d'activités : endoglucanases, exoglucanases et cellobiases, ces dernières étant également appelées β glucosidases. Les enzymes hémicellulolytiques présentent notamment des activités xylanases.

L'hydrolyse enzymatique est efficace et s'effectue dans des conditions douces. En revanche, le coût des enzymes reste très élevé, représentant de 20 à 50% du coût de transformation du matériau ligno-cellulosique en éthanol. De ce fait, beaucoup de travaux ont été conduits pour réduire ce coût : l'optimisation de la production d'enzymes d'abord, en sélectionnant les microorganismes hyperproducteurs et en améliorant les procédés de production desdites enzymes, la diminution de la quantité d'enzymes en hydrolyse ensuite, en optimisant l'étape de prétraitement, en améliorant l'activité spécifique de ces enzymes, et en optimisant la mise en oeuvre de l'étape d'hydrolyse enzymatique.

L'étape de production du cocktail enzymatique comprend trois grandes phases : une phase (a) de croissance d'un microorganisme cellulolytique ; une phase (b) de production du cocktail enzymatique et une phase (c) de séparation et de concentration, au cours de laquelle le cocktail enzymatique est séparé du microorganisme cellulolytique et concentré. La séparation entre le cocktail enzymatique et le microorganisme cellulolytique est effectuée par séparation liquide/solide (centrifugation par exemple). La concentration du cocktail enzymatique est effectué par filtration (ultrafiltration par exemple).

Ces phases de séparation sont nécessaires car le microorganisme cellulolytique, laissé en présence du cocktail enzymatique et en l'absence de substrat carboné, risque de consommer les enzymes contenues dans ledit cocktail afin d'assurer sa survie, résultant ainsi en une perte de production.

De plus, quel que soit le soin apporté à la séparation, il y a toujours une partie du cocktail enzymatique qui est perdue avec la fraction contenant le microorganisme. Suivant les technologies de séparation retenues, ces pertes peuvent correspondre à 3 à 50% poids du cocktail enzymatique présent avant la séparation. Il apparait donc intéressant d'utiliser le moût sans séparation pour éviter ces pertes.

Les températures de fonctionnement d'une étape de SSF sont de l'ordre de 30 à 35 °C. Or ces températures sont compatibles avec la croissance des microorganismes cellulolytiques. Donc si on envisage d'utiliser le cocktail enzymatique dans une étape de SSF sans séparation du microorganisme cellulolytique, il y a un risque de compétition entre le microorganisme cellulolytique et la levure utilisée pour la fermentation, résultant dans une diminution du rendement de ladite étape de SSF. En effet, une partie de glucose libéré par hydrolyse serait consommé par le microorganisme cellulolytique.

Il est donc intéressant de parvenir à désactiver le microorganisme cellulolytique de manière à ne pas avoir à procéder à l'étape de séparation du cocktail enzymatique et dudit microorganisme cellulolytique en vue de son stockage et/ou de son utilisation dans une étape de SSF. Il est également intéressant de parvenir à désactiver ce microorganisme en utilisant un moyen interne au procédé, par exemple un produit d'une des étapes du procédé.

GB1489145 enseigne la culture du microorganisme cellulolytique et d'enzymes à partir de résidus de cellulose, ainsi que l'utilisation de l'ensemble du milieu de culture/production des enzymes sans séparation d'aucun constituant pour l'hydrolyse enzymatique. Ils affirment que l'utilisation de l'ensemble du milieu « selon l'invention », sans traitement (filtration, concentration ou autre) autre qu'un éventuel ajustement de pH, permet d'améliorer l'hydrolyse de la cellulose en terme de vitesse et de rendement.

Ce brevet ne dit rien du problème de croissance potentiel du microorganisme cellulolytique T.reesei dans les conditions de l'hydrolyse enzymatique, ni de la reconsommation des protéines par l'organisme cellulolytique. En effet l'hydrolyse enzymatique est réalisée en général à des températures qui empêchent la croissance du microorganisme cellulolytique (entre 45 °C et 55 °C) contrairement aux SSF qui sont réalisées à des températures comprises entre 30 et 35 °C.

Barta et al. (« Process Design and Economics of On-Site Cellulase Production on Various Carbon Sources in a Softwood-Based Ethanol Plant », Enzyme Research, Vol.2010, doi :10.4061/2010/734182) divulguent l'addition de la totalité du milieu contenant le microorganisme cellulolytique à l'étape de SSF. Selon les auteurs, ceci ne pose pas de problème car la SSF est conduite à 37°C, alors que la croissance du microorganisme cellulolytiqueest totalement inhibée lorsque la température dépasse 35°C. Ce postulat est largement sujet à caution, certaines espèces de Trichoderma (*T. pseudokoningii*, *T*. *saturnisporum* par exemple) étant connues pour être capables de se développer jusqu'à 40-41 °C.

Or les travaux de la demanderesse l'on conduit à découvrir que certains flux internes au procédé de conversion biochimique de la biomasse en éthanol permettait la désactivation du microorganisme cellulolytique.

La présente invention propose un procédé permettant la désactivation du microorganisme cellulolytique levant les inconvénients existant jusqu'à présent.

### RÉSUMÉ ET INTÉRÊT DE L'INVENTION

En désactivant le microorganisme cellulolytique par mise en contact avec un flux gazeux interne au procédé de conversion biochimique de la biomasse en éthanol contenant moins de 0,5% molaire d'oxygène, on supprime la nécessité de séparer ledit microorganisme du cocktail enzymatique produit.

Le choix judicieux du flux gazeux interne utilisé permet d'éviter tout impact négatif sur les performances du procédé pouvant être provoqué par l'accumulation d'espèces indésirables par effet de recyclage.

Un avantage du procédé selon l'invention est donc de limiter les pertes en cocktail enzymatique produit. Un autre avantage est de limiter la taille des installations : en limitant les pertes, on limite la surproduction nécessaire à la compensation desdites pertes. Un autre avantage est de limiter les rejets, en minimisant, voire en supprimant, les phases de nettoyage lors des séparations cocktail enzymatique/microorganisme cellulolytique. Un autre avantage est de minimiser les risques de contamination en limitant, voire en supprimant, des phases de séparation cocktail enzymatique/microorganisme cellulolytique.

Les avantages de la présente invention, énumérés ici de façon non exhaustive, permettent de réduire le coût de production de l'éthanol ex matériaux ligno-cellulosiques et participent à la compétitivité de cette filière.

### DESCRIPTION DÉTAILLÉE DE L'INVENTION

L'invention concerne un procédé de désactivation d'un microorganisme cellulolytique permettant la production d'un cocktail enzymatique, ledit cocktail étant utilisé sans séparation du microorganisme cellulolytique lors de la conversion biochimique de matériaux ligno-cellulosiques, comprenant au moins une étape de mise en contact d'un flux gazeux et du milieu contenant ledit microorganisme, ledit flux gazeux comprenant plus de 25 % massique de CO₂ et comprenant moins de 0,5% molaire d'O₂.

Préférentiellement, ledit flux gazeux a pour origine une étape de SSF.

Préférentiellement, ledit flux gazeux a subit un traitement visant à réduire d'au moins 25% sa teneur en éthanol et en composés organiques volatils avant l'étape de mise en contact.

Préférentiellement, ledit traitement est un lavage à l'eau dudit flux gazeux.

Préférentiellement, ladite étape de mise en contact est réalisée dans une étape de SSF.

Préférentiellement, ladite étape de mise en contact est réalisée dans l'étape de production du cocktail enzymatique, à l'issue de la phase de production dudit cocktail.

Préférentiellement, ladite étape de mise en contact est réalisée entre l'étape de production du cocktail enzymatique et une étape de SSF. Le microorganisme cellulolytique est choisi parmi les souches de champignons appartenant aux genres Trichoderma, Aspergillus, Penicillium ou Schizophyllum.

Préférentiellement, le microorganisme cellulolytique appartient à l'espèce Trichoderma reesei.

L'étape de production du cocktail enzymatique met en oeuvre un microorganisme cellulolytique. Ledit procédé de production est mené en culture submergée. Par culture submergée, on entend une culture en milieu liquide.

Les microorganismes cellulolytiques mis en oeuvre dans le procédé de production d'un cocktail enzymatique sont des souches de champignons appartenant aux genres *Trichoderma, Aspergillus, Penicillium* ou *Schizophyllum*, de préférence appartenant à l'espèce *Trichoderma reesei*. Les souches industrielles les plus performantes sont les souches appartenant à l'espèce *Trichoderma reesei,* modifiées pour améliorer le cocktail enzymatique par les procédés de mutation-sélection, comme par exemple la souche IFP CL847 (brevet français FR-B-2 555 803). Les souches améliorées par les techniques de recombinaison génétique peuvent être également utilisées. Ces souches sont cultivées en réacteurs agités et aérés dans des conditions compatibles avec leur croissance et la production des enzymes.

Conformément à l'invention, on procède donc à la mise en contact d'un flux gazeux comprenant moins de 0,5% molaire d'oxygène (O₂), préférentiellement moins de 0,25% molaire, et plus préférentiellement exempt d'oxygène avec le milieu contenant ledit microorganisme cellulolytique.

La fermentation alcoolique est une réaction biochimique au cours de laquelle des organismes, par exemple la levure *Saccharomyces cerevisiae*, transforment des sucres en éthanol et en dioxyde de carbone (CO₂).

Un flux gazeux comprenant le CO₂ produit lors de l'étape de fermentation alcoolique et/ou lors de l'étape de SSF, est séparé au cours de la fermentation, par dégazage continu du fermenteur. Un flux gazeux comprenant la fraction de CO₂ dissoute dans le milieu de fermentation est produit lors de l'étape de purification de l'éthanol.

Le flux gazeux utilisé dans la présente invention est avantageusement constitué d'un mélange du flux gazeux séparé par dégazage continu du fermenteur et du flux produit lors de l'étape de purification de l'éthanol.

Le flux gazeux produit lors de l'étape de fermentation alcoolique comprend au moins 25% massique de CO₂, préférentiellement au moins 50% massique, et plus préférentiellement au moins 75% massique. Ledit flux gazeux comprend également entre 0 et 10% poids d'éthanol, préférentiellement entre 0 et 5% poids. Ledit flux gazeux comprend également de l'eau et des composés organiques volatils (COV), ces derniers étant définis selon l'article 2 de la directive 1999/13/CE du Conseil du 11 mars 1999.

Lors de ladite étape de purification de l'éthanol, ladite fraction de CO₂ dissoute est séparée du milieu contenant l'éthanol par tout moyen connu de l'homme du métier, par exemple par détente flash, qui consiste à abaisser la pression du milieu pour vaporiser le CO₂ dissout, par distillation, par séparation membranaire, ou par combinaison de ces moyens ou d'autres moyens connus de l'homme du métier.

De manière préférée, le flux gazeux contenant le CO₂ séparé par dégazage continu et/ou le flux gazeux contenant le CO₂ séparé dans l'étape de purification de l'éthanol est traité dans une étape d'abattage de manière à réduire sa teneur en éthanol et en COV ainsi que, si nécessaire, sa teneur en oxygène. Ladite étape d'abattage peut être effectuée par toute méthode connue de l'homme du métier. Préférentiellement, ladite étape d'abattage est un lavage à l'eau ou une séparation membranaire.

Ladite étape d'abattage vise à réduire de 25 à 100% la teneur en éthanol et en COV dans le flux gazeux, préférentiellement de 50 à 100% et plus préférentiellement de 75 à 100%.

Dans un arrangement préféré, le flux gazeux est mis en contact avec le milieu contenant le microorganisme cellulolytique sans traitement préalable dans ladite étape d'abattage.

Lors de ladite mise en contact, l'éthanol éventuellement contenu dans ledit flux gazeux est absorbé dans ledit milieu contenant le microorganisme cellulolytique. Par effet de recyclage, on aboutit ainsi à une concentration de l'éthanol accrue à l'issue de l'étape de fermentation alcoolique. Cet accroissement de la concentration en éthanol a pour effet de diminuer la consommation énergétique de l'étape de purification de l'éthanol.

Lors de ladite mise en contact, le CO₂ va acidifier le milieu. La mise en contact du flux gazeux et du milieu contenant le microorganisme cellulolytique se fera toujours sous contrôle, et éventuellement régulation, du pH.

La mise en contact du flux gazeux et du milieu contenant le microorganisme cellulolytique, ledit milieu ayant un comportement similaire à un liquide, peut être effectué par tout moyen de mise en contact intime d'un gaz et d'un liquide connu de l'homme du métier. De tels moyens sont décrits par exemple, de manière non exhaustive, dans « Adsorption en traitement d'air », Michel Roustan, Techniques de l'ingénieur, G1750.

De manière préférée, l'étape de mise en contact du flux gazeux avec le milieu contenant le microorganisme cellulolytique est réalisée dans une étape de SSF. Pour réaliser cette mise en contact, l'équipement dans lequel est mise en oeuvre une étape de SSF est équipé d'un système de dispersion de gaz dans le milieu liquide. Une autre méthode consiste à extraire une fraction du milieu réactionnel contenu dans ledit équipement, de mélanger intimement ladite fraction avec ledit flux gazeux et de réintroduire ladite fraction, débarrassée ou non le la partie gazeuse dans ledit équipement. Une autre méthode consiste à injecter ledit flux gazeux dans le ciel gazeux dudit équipement.

De manière préférée, l'étape de mise en contact du flux gazeux avec le milieu contenant le microorganisme cellulolytique est réalisée lors de l'étape de production du cocktail enzymatique, à l'issue de la phase (b) de production dudit cocktail. L'équipement permettant la production du cocktail enzymatique étant, comme il est connu de l'homme du métier et décrit par exemple dans le brevet EP 1 690 944, aéré et agité, on coupe l'injection d'air à l'issue de ladite phase (b) de production et on injecte en lieu et place ledit flux gazeux jusqu'à ce qu'on ait une pression partielle en oxygène inférieure à 0,5% molaire, préférentiellement inférieure à 0,25% molaire et plus préférentiellement de 0%. Un autre avantage de l'ajout de CO₂ est de permettre de baisser le pH préférentiellement entre 3,5 et 3,7. Ces pH bas permettent de limiter les risques de contaminations par rapport au pH de culture qui est compris entre 4,8 et 4. On fera attention à cette étape à ne pas descendre à un pH inférieur à 3,3 qui pourrait avoir un effet négatif sur l'activité de certaines enzymes du cocktail.

De manière préférée, l'étape de mise en contact du flux gazeux avec le milieu contenant le microorganisme cellulolytique est réalisée entre l'étape de production du cocktail enzymatique et l'étape de SSF. Cette mise en contact peut être réalisée dans un équipement dédié mettant en oeuvre les technologies classiques de mise en contact gaz/liquide connues de l'homme du métier.

Le flux gazeux contenant le CO₂ peut également être utilisé pour la production de microalgues destinées à la production de biocarburant dit « de troisième génération » après extraction des lipides desdites microalgues, permettant ainsi de produire un co-produit à forte valeur ajoutée, soit directement pour faire de l'aquaculture.

D'autres flux gazeux comprenant moins de 0,5% molaire d'oxygène peuvent également être utilisés, par exemple les effluents des unités de traitement anaérobie de l'eau, les fumées de combustion des déchets, les effluents des unités de méthanation des déchets organiques.

Les exemples qui suivent illustrent l'invention sans en limiter la portée. Dans ce qui suit, l'activité du cocktail enzymatique est mesurée en Unités Papier Filtre et notée FPU (Filter Paper Unit selon la terminologie anglo-saxonne). Cette activité est mesurée sur papier Whatman n° 1 (Procédure recommandée par la commission biotechnologique IUPAC) à la concentration initiale de 50 g.L-1 ; on détermine la prise d'essai de la solution enzymatique à analyser qui libère l'équivalent de 2 g.L-1 de glucose (dosage colorimétrique) en 60 minutes.

### Exemple 1 : Comparaison de conservations de moût de Trichoderma reesei

*L'exemple 1 présente l'évolution de l'activité du cocktail enzymatique d'un milieu contenant à la fois ledit cocktail enzymatique et le microorganisme cellulolytique T.reesei, appelé moût, conservé durant 3 semaines et ayant subi trois traitements différents. Cet exemple montre que l'activité FPU est conservée dans le moût qui a été mis sous atmosphère de CO₂ ou de N₂ alors qu'elle diminue fortement dans celui conservé sous air*.

Une production d'un cocktail enzymatique par *Trichoderma reesei* CL847 est réalisée en fermenteur selon un protocole classique tel que décrit, par exemple, dans la demande de brevet EP 1690944. La concentration en cocktail enzymatique obtenue est de 39.9 g/L et l'activité est de 32 FPU/mL.

Le moût est partagé, sans séparation entre le microorganisme cellulolytique et le surnageant contenant le cocktail enzymatique, dans différentes fioles préalablement stérilisées.

Dans la fiole A, on fait buller un flux gazeux comprenant plus de 99% molaire de CO₂ et contenant moins de 0,5% molaire d'O₂ jusqu'à ce que le ciel gazeux contienne moins de 0,5% molaire d'O₂. Ledit flux gazeux a été obtenu en récoltant le produit de dégazage d'une fermentation alcoolique réalisée par ailleurs.

Dans la fiole B, on fait buller un flux gazeux comprenant plus de 99% molaire de N₂ et contenant moins de 0,5% molaire d'O₂ jusqu'à ce que le ciel gazeux contienne moins de 0,5% molaire d'O₂.

Dans la fiole C, on conserve le moût sous air, dans une fiole stérile avec filtre de 0.2µm.

Le % d'O₂ est mesuré par Chromatographie Phase Gaz (CPG) sur un échantillon prélevé dans le ciel gazeux.

Les fioles sont ensuite conservées à 33 °C durant 3 semaines.

Les activités obtenues après trois semaines sont reportées dans le tableau 1. Plus de 90% de l'activité est maintenue sous atmosphère de CO₂ ou d'azote alors que 50% de l'activité est perdue lorsque le champignon est en présence d'air. Le microorganisme cellulolytique est donc bien inactivé en l'absence d'air.

**Tableau 1 Évolution des activités enzymatiques**

| | Activité initiale FPU/mL | Activité après trois semaines FPU/mL |
|---|---|---|
| Fiole A | 32,00 | 29,08 |
| Fiole B | | 30,44 |
| Fiole C | | 15,15 |

### Exemple 2: Comparaison de deux SSF

*L'exemple 2 compare deux SSF réalisées à 33 °C en utilisant un moût contenant à la fois le microorganisme cellulolytique et le cocktail enzymatique avec ou sans ajout de CO₂ dans le ciel gazeux*.

Deux SSF sont réalisées en utilisant directement le moût de production d'enzymes (cocktail enzymatique+microorganisme cellulolytique) dans le réacteur d'hydrolyse et de fermentation avec une dose d'enzyme de 30 mg d'enzymes par gramme de Matière Sèche (MS). Le pourcentage poids de matière sèche est le ratio de masse de l'échantillon obtenue après séchage à 105 °C pendant 24 heures sur la masse initiale de l'échantillon. La masse de matière sèche est le produit du pourcentage poids de matière sèche et du poids de l'échantillon.

L'expérience est réalisées à 18 % de MS (paille de blé explosée à la vapeur en condition acide, lavée et séchée) dans un réacteur de 2 L. La température est régulée à 33 °C et le pH est régulé à 5 par de la soude (NaOH) à 5 N. La levure est ajoutée 1 heure après le démarrage de l'hydrolyse à une concentration de 0,5 g de levure par kg de milieu de fermentation. La première SSF (SSF1) est réalisée avec un léger bullage d'un flux gazeux comprenant 99,8% molaire de CO₂ et 0,2% molaire d'O₂. La seconde SSF (SSF2) est réalisée sans bullage de CO₂.

SSF1 a abouti à une concentration finale en éthanol de 40,2 g/L alors que SSF2 a abouti à une concentration finale de seulement 34,1 g/L. Le rendement de SSF1 est supérieur de 17,9% à celui de SSF2. Dans le cas de SSF2, le microorganisme cellulolytique a consommé une partie du sucre libéré par l'hydrolyse de la cellulose, faisant diminuer le rendement final de production d'éthanol.

## Revendications

1. Procédé de désactivation d'un microorganisme cellulolytique choisi parmi les souches de champignons appartenant aux genres Trichoderma, Aspergillus, Penicillium ou Schizophyllum, permettant la production d'un cocktail enzymatique, ledit cocktail étant utilisé sans séparation du microorganisme cellulolytique lors de la conversion biochimique de matériaux ligno-cellulosiques, comprenant au moins une étape de mise en contact d'un flux gazeux et du milieu contenant ledit microorganisme, ledit flux gazeux comprenant plus de 25 % massique de CO₂ et comprenant moins de 0,5% molaire d'O₂.

2. Procédé selon la revendication 1 tel que ledit flux gazeux a pour origine une étape de SSF.

3. Procédé selon l'une des revendications 1 à 2 tel que ledit flux gazeux a subit un traitement visant à réduire d'au moins 25% sa teneur en éthanol et en composés organiques volatils avant l'étape de mise en contact.

4. Procédé selon la revendication 3 tel que ledit traitement est un lavage à l'eau dudit flux gazeux.

5. Procédé selon l'une des revendications 1 à 4 tel que ladite étape de mise en contact est réalisée dans une étape de SSF.

6. Procédé selon l'une des revendications 1 à 5 tel que ladite étape de mise en contact est réalisée dans l'étape de production du cocktail enzymatique, à l'issue de la phase de production dudit cocktail.

7. Procédé selon l'une des revendications 1 à 6 tel que ladite étape de mise en contact est réalisée entre l'étape de production du cocktail enzymatique et une étape de SSF.

8. Procédé selon l'une des revendications 1 à 7 tel que le microorganisme cellulolytique appartient à l'espèce Trichoderma reesei.

## Patentansprüche

1. Verfahren zur Deaktivierung eines cellulolytischen Mikroorganismus, ausgewählt aus den Pilzstämmen, die zu den Gattungen Trichoderma, Aspergillus, Penicillium oder Schizophyllum gehören, das die Herstellung eines Enzymcocktails ermöglicht, wobei der Cocktail ohne Abtrennung des cellulolytischen Mikroorganismus während der biochemischen Umwandlung der Lignocellulosematerialien verwendet wird, umfassend mindestens einen Schritt zum Inkontaktbringen eines Gasstroms mit dem Medium, das den Mikroorganismus enthält, wobei der Gasstrom mehr als 25 Masse-% CO₂ umfasst und weniger als 0,5 Mol-% O₂ umfasst.

2. Verfahren nach Anspruch 1, wobei der Gasstrom aus einem SSF-Schritt stammt.

3. Verfahren nach einem der Ansprüche 1 bis 2, wobei der Gasstrom einer Behandlung unterzogen wurde, um seinen Gehalt an Ethanol und an flüchtigen organischen Verbindungen vor dem Schritt zum Inkontaktbringen um mindestens 25 % zu reduzieren.

4. Verfahren nach Anspruch 3, wobei es sich bei der Behandlung um eine Wäsche des Gasstroms mit Wasser handelt.

5. Verfahren nach einem der Ansprüche 1 bis 4, wobei der Schritt zum Inkontaktbringen in einem SSF-Schritt durchgeführt wird.

6. Verfahren nach einem der Ansprüche 1 bis 5, wobei der Schritt zum Inkontaktbringen in dem Schritt zur Herstellung des Enzymcocktails am Ende der Herstellungsphase des Cocktails durchgeführt wird.

7. Verfahren nach einem der Ansprüche 1 bis 6, wobei der Schritt zum Inkontaktbringen zwischen dem Schritt zur Herstellung des Enzymcocktails und einem SSF-Schritt durchgeführt wird.

8. Verfahren nach einem der Ansprüche 1 bis 7, wobei der cellulolytische Mikroorganismus zur Spezies Trichoderma reesei gehört.

## Claims

1. A process for deactivating a cellulolytic microorganism selected from strains of fungi belonging to the genera Trichoderma, Aspergillus, Penicillium or Schizophyllum, enabling the production of an enzymatic cocktail, said cocktail being used without separating the cellulolytic microorganism during the biochemical conversion of lignocellulosic materials, comprising at least one step for bringing a gaseous stream into contact with a medium containing said microorganism, said gaseous stream comprising more than 25% by weight of CO₂ and comprising less than 0.5 molar % of O₂.

2. The process according to claim 1, in which said gaseous stream originates from a SSF step.

3. The process according to claim 1 or claim 2, in which said gaseous stream has undergone a treatment for reducing its ethanol and volatile organic compounds content by at least 25% before the contact step.

4. The process according to claim 3, in which said treatment is washing said gaseous stream with water.

5. The process according to one of claims 1 to 4, in which said contact step is carried out in a SSF step.

6. The process according to one of claims 1 to 5, in which said contact step is carried out in the enzymatic cocktail production step at the end of the phase for production of said cocktail.

7. The process according to one of claims 1 to 6, in which said contact step is carried out between the enzymatic cocktail production step and a SSF step.

8. The process according to one of claims 1 to 7, in which the cellulolytic microorganism belongs to the species Trichoderma reesei.
